# EUROPEAN PATENT APPLICATION

(11) **EP 4 495 942 A1**
(43) Date of publication of application: **22.01.2025**
(21) Application number: 24189866.7
(22) Date of filing: 19.07.2024
(51) Int. Cl.: G16H 30/20

(54) **MEDICAL IMAGE DATA ANALYSIS AND VISUALIZATION**

(30) Priority: 19.07.2023 US 202363514453 P
(71) Applicant: Hologic, Inc., Marlborough, MA 01752 (US)
(72) Inventor: DSOUZA, Adora, Marlborough, MA 01752 (US); CHUI, Haili, Marlborough, MA 01752 (US); KSHIRSAGAR, Ashwini, Marlborough, MA 01752 (US)
(74) Representative: Hoyng Rokh Monegier B.V.

(57) **Abstract**

A system and method of analysis for medical image data. An image of breast tissue is received, a region of interest (ROI) in the image is identified based on tissue characteristics, and a query image is defined. A hierarchy of lesion data in a lesion database is retrieved, the hierarchy being formed based on one or more relationships among a plurality of images, and the query image is positioned within the hierarchy of lesion data based on the tissue characteristics. A position of the query image within the hierarchy of the lesion data in the lesion database is determined, identifying one or more neighbor images of the query image from among the plurality of images based on the position, and statistics associated the one or more neighbor images are retrieved. Analytics associated with the query image based on the statistics are generated, and a graphic depicting the analytics is displayed.

## Description

### BACKGROUND

During the review process of two-dimensional and three-dimensional medical images, such as mammography images and tomosynthesis images, respectively, a critical task for clinicians is to evaluate an image and determine its probability of representing either a benign or malignant lesion. Based on the evaluation, clinical diagnostic decisions are made. For example, highly suspicious findings are biopsied, less suspicious findings can be monitored through follow-up scans, and the least suspicious, or benign, findings are dismissed.

### SUMMARY

Examples presented herein relate to a method of analysis for medical image data. The method includes receiving an image of breast tissue and identifying at least one region of interest (ROI) in the image based on one or more tissue characteristics associated with ROI. The method further includes defining a query image as a subset of the image of breast tissue including the ROI, retrieving a hierarchy of lesion data from a lesion database including a plurality of images of breast tissue, the hierarchy being formed based on one or more relationships among the plurality of images, and positioning the query image within the hierarchy of lesion data based on the one or more tissue characteristics. The positioning of the query image can include or can be replaced by determining a position of the query image within the hierarchy of the lesion data in the lesion database. The method can further include identifying one or more neighbor images of the query image from among the plurality of images based on the position. The method can further include retrieving statistics associated with the one or more neighbor images. The method further includes generating analytics associated with the query image based on the statistics, generating a graphic depicting the analytics, and displaying the graphic.

In other examples presented herein, the hierarchy of lesion data is retrieved based on a selection received from a user. In still other examples presented herein, the hierarchy of lesion data is based on a pre-generated archive. In yet other examples presented herein, the hierarchy of lesion data is based on an image generated neural network. In further examples presented herein, the hierarchy of lesion data is based on a personalized archive associated with the one or more users. In other further examples presented herein, the selection comprises parameters defining a subset of the plurality of images.

In other examples presented herein, the hierarchy of lesion data in the lesion database includes the subset of the image containing the ROI. In still other examples presented herein, the hierarchy of lesion data further comprises one or more of patient information, semantic characteristics, and analytical characteristics. In yet other examples presented herein, the hierarchy includes a high dimensional hierarchical statistical graph.

In further examples presented herein, the one or more relationships are based on a first set of features used to classify the plurality of medical images. In other further examples presented herein, the first set of features defines a first axis upon which the hierarchy is structured. In still further examples presented herein, a second set of features defining a second axis; wherein the hierarchy is structured upon both the first axis and the second axis. In yet further examples presented herein, a second set of features used to classify the plurality of medical images, wherein classifications based on the second set of features are indicated using a range of colors.

In other examples presented herein, the graphic is generated based on a selection received from a user, wherein the selection indicates one or more of a horizontal network graph, a radial graph, a hierarchical cluster tree, a two-dimensional graphic, a three-dimensional graphic, and a graphic depicting on a subset of hierarchy. In still other examples presented herein, the graphic of the analytics includes one or more of the hierarchy of lesion data, the one or more neighbor images, and the statistics.

In other examples presented herein, the method further includes generating a time progression of the query image based on the one or more neighbor images. In yet other examples presented herein, the method further includes generating a projection mapping a trajectory of the query ROI's through a multi-dimension space. In still other examples presented herein, the query image is associated with a first imaging modality, and the method further includes generating a depiction of the query image associated with a second imaging modality based on the one or more neighbor images. In further examples presented herein, the time progression or the image from the second imaging modality is generated using an image generation neural network.

In other examples presented herein, the statistics includes one or more of: a recalled number of lesions in the one or more neighbor images, a biopsied number of lesions in the one or more neighbor images, a malignant-identified number of lesions in the one or more neighbor images, a margin measurement, a shape measurement, a risk measurement, a density measurement, a size measurement, a characterization of morphology, a characterization of pathology, and a characterization of functional properties including at least elasticity, stiffness, and angiogenesis. A variety of additional inventive aspects will be set forth in the description that follows. The inventive aspects can relate to individual features and to combinations of features. It is to be understood that both the forgoing general description and the following detailed description are exemplary and explanatory only and are not restrictive of the broad inventive concepts upon which the embodiments disclosed herein are based.

### BRIEF DESCRIPTION OF THE DRAWINGS

The accompanying drawings, which are incorporated in and constitute a part of the description, illustrate several aspects of the present disclosure. A brief description of the drawings is as follows:
FIG. 1 illustrates an example network environment in which aspects of the present disclosure may be implemented.
FIG. 2 illustrates a block diagram of an example medical image analysis and visualization system.
FIG. 3 illustrates an example structure for the structured lesion database.
FIG. 4 illustrates another block diagram of an example medical image analysis and visualization system.
FIG. 5 illustrates a flowchart an example method 400 of constructing a hierarchical data structure.
FIG. 6 illustrates a flowchart of an example method for using the analysis and visualization system to evaluate a query image.
FIG. 7 illustrates a block diagram of the example method of FIG. 6.
FIG. 8 illustrates another example visualization in the form of a hierarchical cluster tree.
FIG. 9 illustrates another example visualization in the form of a radial graph.
FIG. 10 illustrates a flowchart of an example method 600 of defining inference engine s based on clinical tasks.
FIG. 11 illustrates an example computing system with which aspects of the present disclosure may be implemented.

### DETAILED DESCRIPTION

Aspects of the present disclosure relate to systems and methods for automated analysis of medical image data and generating visualizations to make analytical results more accessible to clinicians and other users. Reference will now be made in detail to exemplary aspects of the present disclosure that are illustrated in the accompanying drawings. Wherever possible, the same reference numbers will be used throughout the drawings to refer to the same or like parts.

Medical image analysis is central to many current diagnostic determinations, such as the presence or absence of cancer or other diseases. While a number of tools have been developed in recent years to standardize and otherwise assist in this analysis, it remains a substantially subjective process. Each clinician must rely on their own knowledge and prior experience in making diagnostic determinations.

At least two factors contribute significantly to the difficulty of the clinical diagnostic decision process. The first is the low occurrence rate of many cancers. Even with the latest advancements in medical imaging technologies, it is still expected that an average radiologist will only find approximately five or six cancers in a screening population pool of 1000 patients. It takes a clinician a significant portion of their career to reach a statistically large scale of observed cancers in their internal "archive" that they can draw information from for diagnosis. The second factor is the variability between clinicians and other medical image data readers. Cancerous and other disease lesions come in many different types, shapes, and characteristics, and are frequently obscured by adjacent or nearby tissue. Diagnostic decisions based on medical image data thus continue to be highly subjective, and significant degrees of difference have been recorded between different readers based on common image data.

Disclosed herein are a medical image analysis system and methods to address these challenges. The disclosed system uses large-scale medical image data including examples of diverse clinical characteristics and outcomes. Using this large-scale medical image data, the disclosed system builds a holistic statistical representation for the different types of lesions. Further disclosed herein are methods of building the holistic statistical representation for different types of lesions. Systems and methods disclosed herein provide intuitive visualization interfaces and tools to help clinicians perform the diagnostic evaluation in an objective statistics-driven manner based on the large-scale medical image data and the holistic statistical representation.

Systems embodying the present disclosure augment a clinician's ability to leverage a large-scale medical image database for evaluating new images objectively for the presence or absence of disease-indicating lesions. Aspects of the present system are interactive, allowing clinicians and other users to explore the holistic statistical representation, which may be a hierarchical graph or map of relationships among analyzed lesions within the large-scale medical image data. Interactive exploration of the map, which may be populated with hundreds, thousands, or more medical images with identified lesions and other regions of interest (ROIs), enables a clinician to visually perceive how different types of lesions are distributed in relation to each other within the high-dimensional, hierarchical statistical map. As discussed herein, lesions refer to images, or portions or subsets of images, that appear anomalous. ROIs broadly refer to images, or portions or subsets of images, that warrant further or more robust inspection.

Disclosed systems and methods herein may enable the extraction of further insight, the discovery of deeper connections between different cases, and the formation of new knowledge about cancer and other disease patterns and progress. The disclosed system may further be applied as a teaching tool for trainee clinicians who are relatively new to studying medical image data and may be unfamiliar with what to look for in identifying a significant lesion.

Disclosed herein is a data representation and visualization system including an interactive statistical network graph of medical image data and lesions. In aspects, the disclosed system may use one or more deep neural networks to evaluate medical image data and construct the holistic statistical representation of the analyzed medical image data. In examples, training of the neural networks may be automated. Training may be cloud-based or otherwise disturbed across a global or other large-scale system, or may be localized on a local server, such as a hospital system, with the data being geographically contained within the local system. In some cases, an archive specific to the local hospital system may be a personalized, location-specific, archive available to users of the local server as a pre-generated archive. In some examples, a location-specific archive may be trained using data acquired using local systems only.

The holistic statistical representation is built upon large-scale medical image and lesion data and may be configured to be highly interactive for clinicians and other users to enable knowledge discovery and decision support. The present disclosure provides for the population and visualization of a map of lesion data and relationships among lesions within the lesion data. The map can be populated using data and deep learning systems to assist clinicians in performing clinical tasks and addressing diagnostic problems. In examples discussed herein, systems and methods may be applied in the particular context of medical image analysis, and more particularly in the context of medical image analysis of breast tissue.

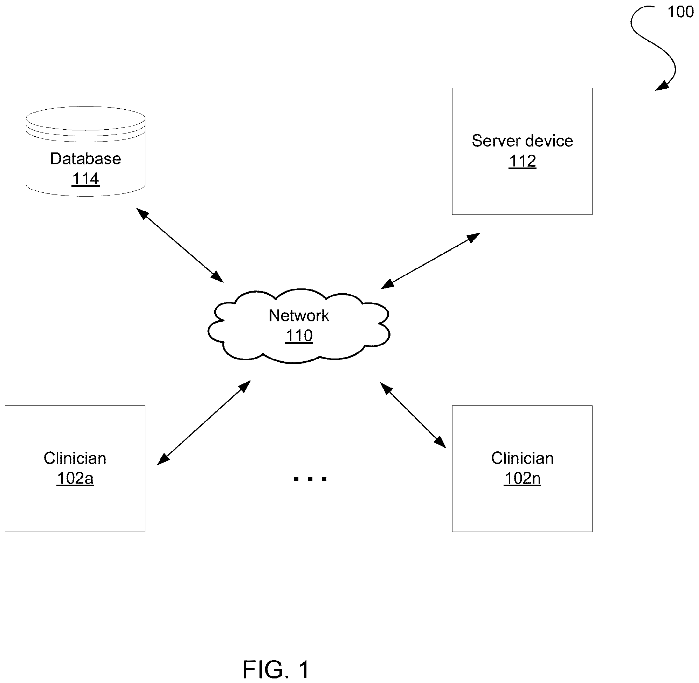

FIG. 1 illustrates an example network environment 100 in which aspects of the present disclosure may be implemented. In particular, the environment 100 may be used to provide analytical and diagnostic tools to clinicians for evaluating medical image data and making diagnostic determinations. The analytical and visualization tools can include machine learning statistics and assembly of a holistic statistical representation of analyzed medical image data.

In the example shown, a medical image analysis server 112 may be communicatively connected to a structured lesion repository server or database 114 via a network 110, such as the Internet. Medical image analysis server 112 may host one or more analysis programs, such as machine learning models and/or relationship-fitting analysis programs, which may be applied to medical image data and holistic statistical data from structured lesion database 114 for purposes of analysis and prediction.

One or more clinicians, via clinician computing devices 102a-n (collectively referred to as clinicians 102 herein, for simplicity), may access one or more diagnostic tool sets provided by medical image analysis server 112, for example to position a query image within a hierarchal map of medical images stored in structured lesion database 114. A query image, as discussed herein, refers to an image submitted into the system for analysis and placement within the lesion database. The query image may be submitted in any number of known image formats and, in embodiments, may be in a medical image format. For example, the query image may be a mammography image, an ultrasound image, a tomosynthesis image, or another medical imaging modality. In embodiments, the query image may be submitted as an entire image or set of images, or may be a portion or subset of a larger overall image or set of images.

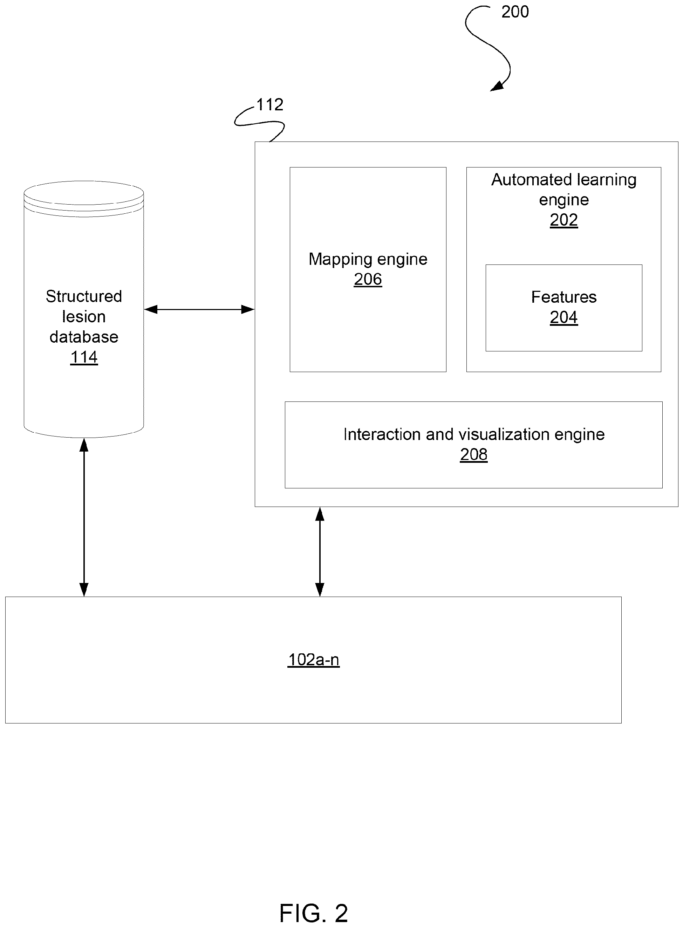

FIG. 2 illustrates a block diagram of an example medical image analysis and visualization system 200. Image analysis and visualization system 200 may include a structured lesion database 114, an inference engine 202, features 204, mapping engine 206, and an interaction and visualization engine 208. Together these components enable the construction and control of structured lesion database 114 using medical image data. The system 200 further provides for analysis of medical image data by inference engine 202, to determine feature 204 for the population of images within the structured lesion database 114 by mapping engine 206. The system 200 provides interactive user access to the structured lesion database 114 through interaction and visualization engine 208 and generates statistical graphics and other visualizations to convey inferences based on the analyzed medical image data to a user to support diagnostic and other clinical analysis of medical images by clinicians.

Structured lesion database 114 stores a carefully constructed data structure of medical image data, lesions, and relationships between different images and lesions. Medical image data including a multitude of medical images is analyzed to identify and characterize lesions within the medical images. The analysis also includes defining relationships among the characterized lesions. Each image of the multitude of medical images or, in examples, each individual lesion identified among the multitude of medical images, may then be arranged within the structured lesion database 114 according to the defined relationships. This arrangement produces a relationship-based map of the data within structured lesion database 114. In examples, the relationship-based map may be configured as a hierarchy. A clinician may trace a query lesion within the hierarchy and evaluate it with the statistics of similar neighboring lesions.

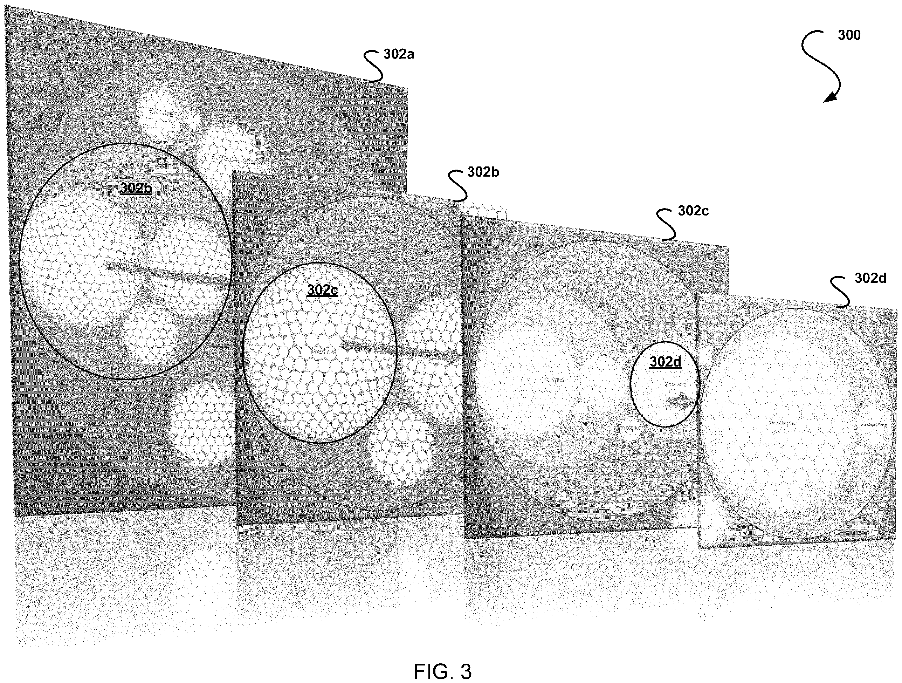

FIG. 3 illustrates an example structure for structured lesion database 114. Analyzed lesions and their characteristics, as they are arranged within structured lesion database 114, may be represented as a hierarchical graph 300. Lesions may be divided into different types and arranged into levels or tiers 302a-d. Each tier may be further subdivided into different subtypes or subgroups and arranged within the tier with some of all of the subgroups leading to a "lower" or more specific tier.

For example, identified lesions may first be divided among subgroups at a high-level tier 302a, such as by being divided into calcifications or soft tissue. In the example of FIG. 3, high-level tier 302a represents soft tissue classifications. Soft tissue masses 302b can be further sub-divided based on shape, margin characteristics. For example, 302c is a subdivision of soft tissue masses with irregular boundaries. Similarly, calcifications can be represented as another hierarchical graph (not shown).

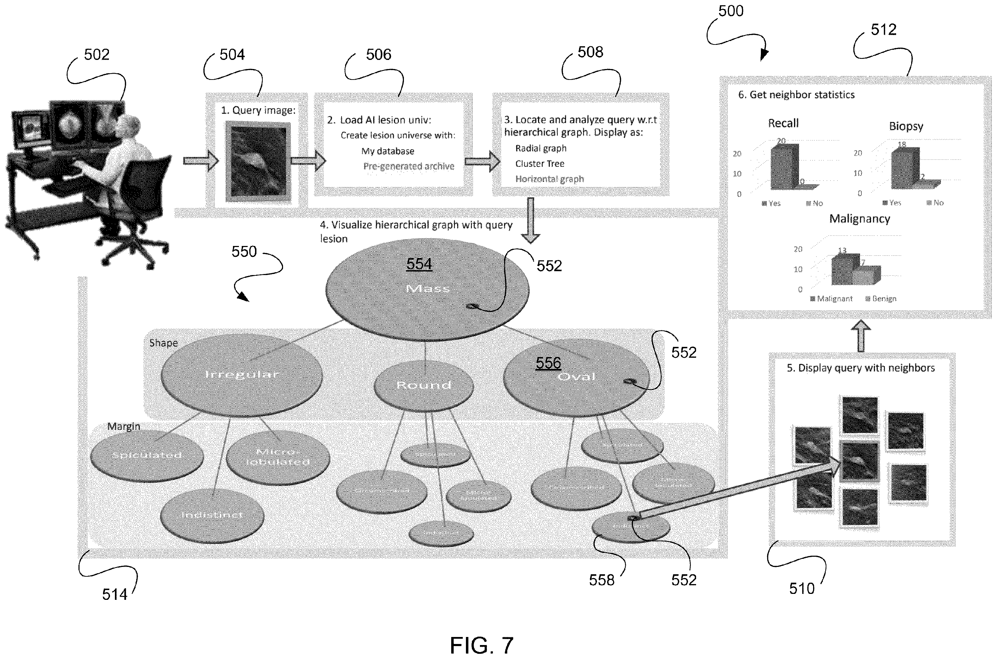

The hierarchical graph 300 can be constructed to contain sematic information associated with lesions, images, or tiers. In the example of FIG. 3, semantic information such as lesion type 302a, shape 302b, margin 302c, and malignancy 302d are shown. However, other characteristics such as size, density, Breast Imaging-Reporting and Data System (BIRADS) score, age, morphology, pathology, functional properties including at least elasticity, stiffness, and angiogenesis, etc., can be used to generate and be reflected in the hierarchical graph 300. Other example structures are contemplated, including those discussed below in relation to FIGS. 7-9. A clinician may choose to view the structed data as one or more of the example structures, or any other hierarchical network representation.

Design and construction of structured lesion database 114 may be defined based on underlying clinical tasks. Clinical tasks may also drive revision, updating, and use of structured lesion database 114. Examples of clinical tasks which may be used to construct the data structure of structure lesion database 114 include evaluating an image for the presence or absence of lesions, evaluating lesions as being benign or malignant, determining attributes associated with a particular lesion or group of lesions, etc.

Data selected to construct structured lesion database 114 may vary based on context or an intended use of analysis and visualization system 200. In examples, medical image data is used to construct structured lesion database 114. Medical image data may be of breast tissue, or another target organ or tissue type. In examples particularly directed to medical image data of breast tissue, data may include mammography images, tomosynthesis images, magnetic resonance images (MRI), ultrasound images, etc.

Numerous organizations or arrangements of data within the structured lesion database 114 are contemplated. In examples, data may be organized according to a single class, such as division between lesion cases and normal cases, where normal cases are those where no lesions are identified as suspicious or requiring further imaging. Data may be classified using nonbinary classifiers, such as classifying among benign lesion cases, malignant lesion cases, and normal cases. Data may be organized according to multiple classes, such as two or more of classifying between lesion cases and normal cases, classifying between benign lesions and malignant lesions, classifying among lesions at different stages, and classifying among lesions with different attributes. A nonlimiting example of a lesion attribute is characterizing a lesion's margin, such as between round lesions and spiculated lesions.

In examples, all data within structured lesion database 114 may be classified, or subsets may be identified, and only one or more subsets subject to some or all of the classifications. For example, medical image data may be classified as lesions cases and normal cases, with lesions cases forming a lesion subset and normal cases forming a normal subset. Only those cases within the lesion subset may then be classified between benign cases and malignant cases.

Inference engine 202 provides one or more learning algorithms or models suitable to define high dimensional features representing identified lesions based on a target clinical task. Features 204 are learned features generated by inference engine 202, their associated feature representations, and the feature mappings to extract information from the features and enable visualization of that information. Together, inference engine 202 and features 204 are used by mapping engine 206 to construct and populate structured lesion database 114.

Inference engine 202 may create features 204 using a number of different techniques, including application of various deep learning (DL) techniques. One or more DL neural networks may be designed to accomplish different learning or clinicals tasks, and then trained using an end-to-end fully automated training framework, where relevant features are determined and identified. In embodiments, the training framework includes training data used to train the one or more DL neural networks, as well as various working components, such as libraries and packages, which may be used in the design of the one or more DL neural networks. The training data may be created by storing images, or subsets of images, with regions of interest identified and labeled.

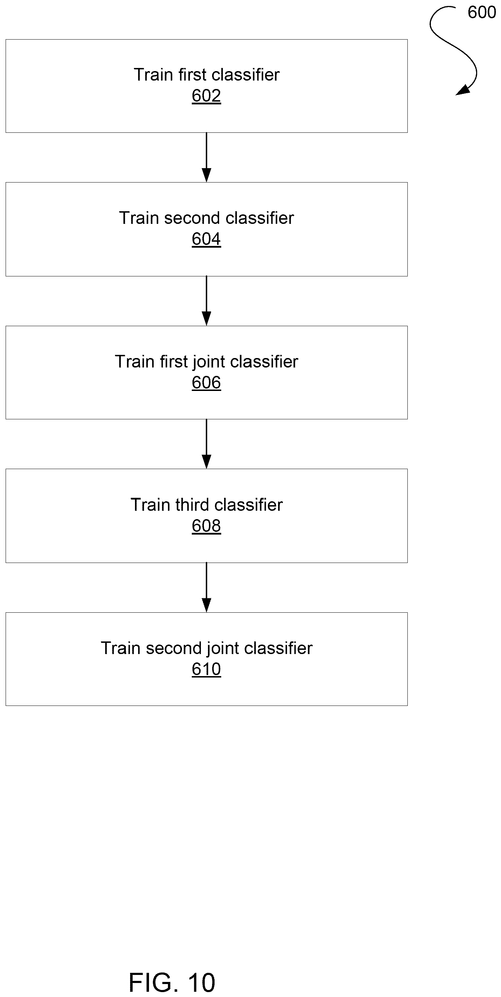

Some non-limiting examples of types of DL neural networks that may be implemented as part of inference engine 202 are classification tasks (such as benign versus malignant), cancer stages, BIRADS scores, shape attributes, cluster groups and lesion similarity. In examples, a classifier can be designed to combine all (or a portion) of these example tasks or other tasks as discussed in further detail below in relation to FIG. 10.

Different clinical and learning tasks can be used to define parameters for inference engine 202 and, as a result, define different features 204. Relevant tasks and associated parameters can be determined by defining data stream. An example task for a single data stream or an individual lesion is classifying the lesion as being malignant or benign, as driven by the clinical task of identifying malignant lesions for further investigation. An example task for multiple data streams, such as a pair of lesions, is classifying the pair as being similar or different, as driven by a learning task of finding ways to organize lesions into meaningful similar and informative classes.

Mapping engine 206 arranges analyzed images and lesions according to features 204 and identified relationships. Mapping engine 206 participates in constructing and organizing structured lesions database 114 and may evaluate new query images or lesions by positioning the query image or lesion within the hierarchical data structure of structured lesions database 114.

The Mapping engine 206 and the features 204 can be combined to directly generate a low dimensional (2D or 3D) representation of lesions using deep learning algorithms that learn cluster information.

Interaction and visualization engine 208 provides components and techniques to enable a user to visualize and interact with structured lesion database 114, inference engine 202, and features 204. Numerous different ways to visualize structured lesion database 114 and features 204 are contemplated, including at least both two-dimensional and three-dimensional visualizations. Interaction and visualization engine 208 may be programmed to enable a user to select a subset of data to visualize. For example, various subsets of data may be informative in different ways to support clinical workflows and provide insight to support clinician decision making.

Interaction and visualization engine 208 may be programmed to receive a query lesion from a user, such as user device 102, and provide visualizations and statistics related to the query lesion based on structured lesion database 114, automated learning 202, and features 204. A query lesion may refer to a lesion identified in medical image data and submitted for analysis based on comparison to lesions and other medical image data arranged in structured lesion database 114.

A query lesion, which is an example of a query image defined as a subset of breast tissue including an identified region of interest (ROI), may be positioned within the hierarchical data structure of structured lesion database 114 by mapping engine 206, and statistics and predictions relevant to the query lesion may be determined based on where the query lesion falls within the hierarchical data structure. In embodiments, the determination of the position within the hierarchy is based on features 204. Other features or properties taken from the query lesion or from the query image or from image of the breast tissue can also be used for determining the position. In further embodiments, meta-data from the query lesion or breast tissue image is used in determining the position. Using suitable properties, features and/or meta data, the query lesion can be positioned within the hierarchical data structure, such that the query lesion is most adjacent or nearest to one or more lesions or medical images that form part of the hierarchical data structure. Once the query lesion is positioned or oriented within the hierarchical data map, these nearest or neighboring lesions and their associated characteristics and outcomes may form a statistical picture of characteristics and predictions relevant to the query lesion. In embodiments, a user may change or modify a query lesions position within the hierarchical data structure. For example, a user may identify that a particular query lesion has been positioned as being malignant but the user knows the particular query lesion is benign. The user may move the query lesion to a benign position within the hierarchical data structure, such as by dragging with a cursor, accessing and revising the labels associated with the particular query lesion, etc.

For example, neighboring lesions may be predominantly classified as either malignant or benign, and may inform the likelihood of malignancy of the query lesion. Neighboring lesions may share a common stage of cancer, or range of stages of cancer, and inform the likely stage or range of stages associated with the query lesions. Neighboring lesions may be stored within structured lesion database 114 with additional data indicating forward and/or backward progression of the lesion over time. This time progression data associated with neighboring lesions may inform construction of a projected timeline for predicted progression of the query lesion. Neighboring lesions may be stored within structured lesion database 114 with additional data depicting the neighboring lesions as imaged by one or more image modalities, e.g., magnetic resonance imaging (MRI), ultrasound, etc. This alternate image modality data associated with the neighboring lesions may inform a prediction of how the query lesion may appear in one or more alternative image modalities.

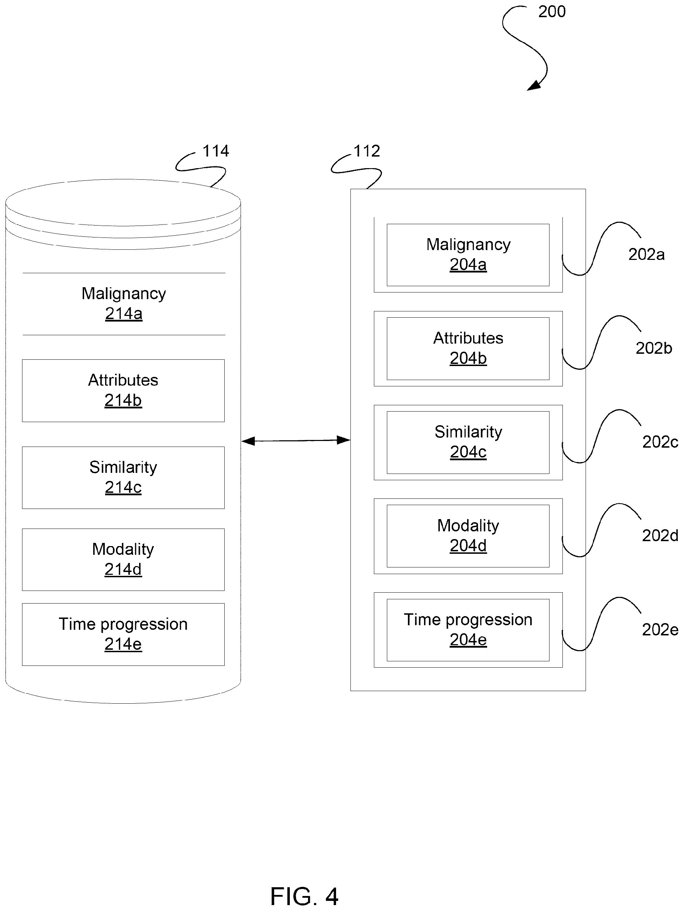

FIG. 4 illustrates another block diagram of an example medical image analysis and visualization system 200 showing additional details of structured lesion database 114 and server device 112. The analysis and visualization system 200 may be constructed using a plurality of databases, 214a-e, which together make up the overall structured lesion database 114. Task- or class-specific databases, such as databases 214a-e may be associated with task- or class-specific features 204a-e and automated learning modules 202a-e. In examples, analysis and visualization system 200 may include a holistic body of data, and various subsets or subdivisions may be accessed by an interaction and visualization component, such as interaction and visualization engine 208 of FIG. 2, based on, for example, input from a user, characteristics of a query lesion, or a target task.

As a reflection of how a clinician forms an understanding of disease and lesions over time through repeated execution of numerous clinical tasks, the analysis and visualization system 200 may generate a task-aligned view of lesion analysis through the various task-specific databases 214a-3, features 204a-e, and learning modules 202a-e. This alignment between traditional analysis and the analysis provided by the system of the present disclosure yields analytics more immediately accessible to clinicians.

FIG. 4 presents a number of non-limiting examples of task-specific components of analysis and visualization system 200. The example tasks that will be discussed in reference to FIG. 4 include evaluating the malignancy of a lesion, characterizing the attributes of a lesion (e.g., margin, density, size, morphology, pathology, etc.), evaluating the similarity or difference among two or more lesions, evaluating the appearance of a lesion using differing imaging modalities (e.g., forming a joint analysis or diagnosis from multi-modality imaging observations), and evaluating the appearance of a lesion over time.

For each task, an inference engine 202a-e, such as a learning algorithm or model, is configured to determine and evaluate a set of features 204a-e relevant to the task. Each inference engine 202a-e may be particularly configured for its specific task. In embodiments, one or more of automated learning modules 202a-e may be configured using a machine learning model, such as a neural network or a deep neural network. Using features 204a-e, a mapping component, such as mapping engine 206 of FIG. 2, evaluates and organizes a database set of medical images, including lesions depicted in the images of the database set of medical images. Images of the database set of medical images are organized into a hierarchical data structure according the task-specific features 204a-e and learning module 202a-e, and stored in a task-specific database 214a-e.

A malignancy inference engine 202a may identify malignancy features 204a associated with a lesion being malignant or benign. Medical image data and associated lesions are organized into a hierarchical data structure according to the malignancy features 204a and stored as a malignancy database 214a. Malignancy database 214a may include all image data entered in structured lesion database 114 organized into a hierarchical data structure according to a degree or outcome of malignancy, or may include only a subset of the image data entered into structured lesion database 114, e.g., normal images, or images without an identified lesion, may be excluded from malignancy database 214a. In examples, malignancy inference engine 202a may be configured as a deep learning neural network classifier model.

An attributes inference engine 202b may identify attribute features 204b associated with characterizing a lesion appearance or other identifying traits. Medical image data and associated lesions are classified according to one or more attribute features 204b and stored as a attributes database 214b. Attributes database 214b may include all image data entered in structured lesion database 114 classified and organized into a data map according to attributes, or may include only a subset of the image data entered into structured lesion database 114. In examples, attributes inference engine 202b may be configured with one or more component modules, such as a semantic neural network model to identify semantic lesion attributes and a segmentation neural network model to segment and measure lesions to determine size and other attributes.

A similarity inference engine 202c may identify similarity and differences features 204c between a pair of lesions or among a larger defined group. Medical image data and associated lesions are organized into a hierarchical data map according to the similarity and differences features 204c and stored as a similarity database 214c. Similarity database 214a may include all image data entered in structured lesion database 114 organized into a hierarchical data map according to similarity, or may include only a subset of the image data entered into structured lesion database 114. In examples, similarity inference engine 202c may be configured as a dual-input deep learning neural network, a triple-input network, a quad-input network, etc.

A modality inference engine 202d may identify modality features 204d associated with a lesion being imaged with a particular medical imaging modality. Modality inference engine 202d provides analysis for multiple modalities, which may be based on a query image taken with a single modality. Inference engine 202d may evaluate an incoming query lesion with neighboring lesions having other imaging modality information (e.g., MRI, ultrasound) and provide information on how the query lesion may appear in the other different modalities. Medical image data and associated lesions are organized into a data map according to the modality features 204d and stored as a modality database 214d. Modality database 214d may include all image data entered in structured lesion database 114 organized into a data map according to modality. Examples of dimensions used to construct modality database 214d may be X-ray images, tomosynthesis images, ultrasounds images, etc. Modality database 214d may be used to construct an image of the query lesion imaged from the different modalities using an image generation neural network. A Generative Adversarial Network (GAN) model is one non-limiting example of an image generation neural network.

A time progression inference engine 202e may identify time progression features 204e associated with a lesion's changing characteristics over time. Time progression inference engine 202e may perform temporal learning and determine features 204e to take static images and generate predictions for multi-point-in-time images. Medical image data and associated lesions are organized into a data map according to the time progression features 204e and stored as a time progression database 214e. Time progression database 214e may include all image data entered in structured lesion database 114 organized into a data map according to modality, e.g., medical image data representing a lesion only imaged once may not contribute to the time progression data map and may be excluded. In examples, time progression inference engine 202e may be configured as a time-sequence neural network model to analyze lesion progress or to compare a current image of a lesion with a prior image of the same lesion. An example of a dimension used to construct time progression database 214e is lesion progression over time.

Time progression database 214e may be used to generate a display, such as a video stream, of neighboring lesion's appearance in previous scans or future scans in place of the original neighboring lesion. Inferences can be drawn based on time progression database 214e, such as a predicted trajectory of the query lesion or a predicted timeline of the query lesion, demonstrating how the lesion may progress over time. An image generation neural network may be used to generate a prediction of how the lesion of interest appears at a future time-point.

As will be understood by those of skill in the art, task-specific learning modules and databases other than those illustrated in FIG. 4 may be incorporated. For example, a stages automated learning module may identify stages features associated with a lesion's disease stage of degree of progression. Image data and identified lesions may be organized into a data structure according to the stage or progression features and stored in a stages database. The stages automated learning module may be configured as a deep learning neural network classifier model. Those of skill in the art will further understand that task may be performed singularly and independently, or multiple different tasks may be performed together. For example, in example implemented automated learning modules 202a-e as neural networks, multiple neural networks may be combined and trained together simultaneously as discussed in greater detail below in relation to FIG. 10.

Other divisions of structured lesion database 114 are contemplated. For example a pre-generated database with particular parameters or image/lesion characteristics; a dynamic database which incorporates each new query image into the structured data map; a database associated with a particular image device or group of devices, such as a group of devices in active use at a particular institution; a clinician may establish a personalized database of known patients; a clinician may request generation and population of a database based on particular parameters or image/lesion characteristics; etc.

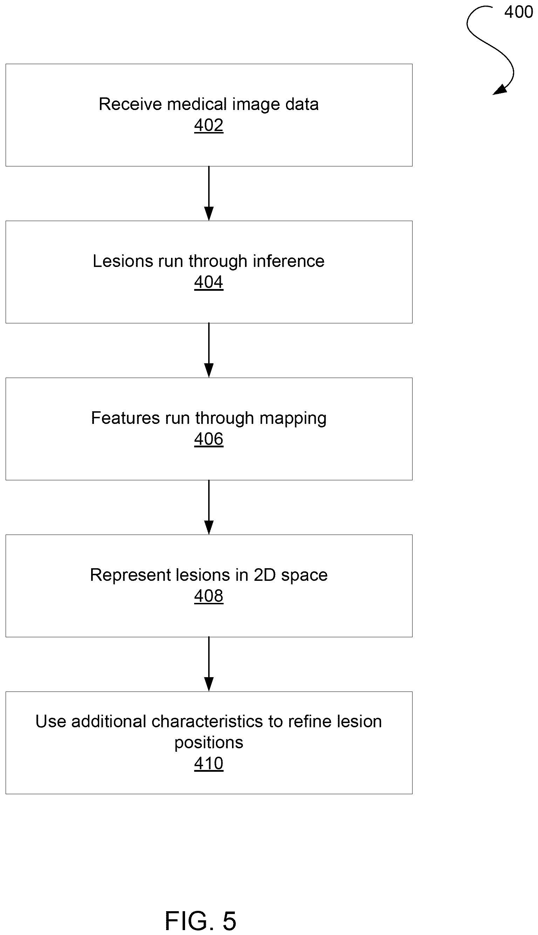

FIG. 5 illustrates a flowchart an example method 400 of constructing a hierarchical data structure. The hierarchical data map structure represents the arrangement of data within structured lesion database 114, a temporary data constructed generated for lesion analysis, or visualizations of lesion analytics and relationships presented to a user.

At 402, medical image data is received. The medical image data may be a pre-generated database of image data or may be generated in response to particular parameters input by a user.

At 404, lesions and other ROIs in the medical image data are run through inference engine to extract features. Lesions generally relate to portions or a subset of the image data which appear anomalous. ROIs may refer more broadly to portions or a subset of the image data for which further inspection is warranted. ROIs may encompass lesions, as well as artifacts and indeterminate areas, as some non-limiting examples.

In examples, feature extraction may be high dimensional, or on the order of hundreds or thousands of dimensions. A high dimensional feature representation is created for each lesion which may encode, for example, a shape of the lesion, the lesion's texture characteristics, surrounding tissue densities, etc.. The high dimensional feature may represent a latent space of the lesion including the defined characteristics. The inference engine may be configured so that feature spaces can be grouped into different categories based on their characteristics, for example, spiculated and irregular masses both falling within a larger group of soft tissue masses. These groupings may organically contribute to producing a hierarchical graph, such as hierarchical graph 300 of FIG. 3.

At 406, the extracted features are mapped to a low dimensional (2D or 3D) space for easy visualization which is to determine one or more relationships among the lesions and other ROIs. At 408, the lesions and other ROIs are arranged by a mapping engine according to their features and relationships to neighboring lesions. The high dimensional representation based on the features obtained from the inference engine may be made into a lower dimensional representation for easier analysis. A two-dimensional (2D) space may be particularly accessible for visualization presented to user, but other lower dimensional representations are also contemplated, including at least three-dimensional (3D) spaces. The generation of a mapping that can project the high dimensional feature representation to a lower dimensional (e.g., 2D or 3D) representation makes interpretation easier, visualizations more straightforward to interact with and more intuitive to manage.

At 410, additional characteristics of lesions may be used to refine lesion positioning within the two-dimensional space. For example, semantic data, such as outcomes and treatments associated with the lesion may be integrated.

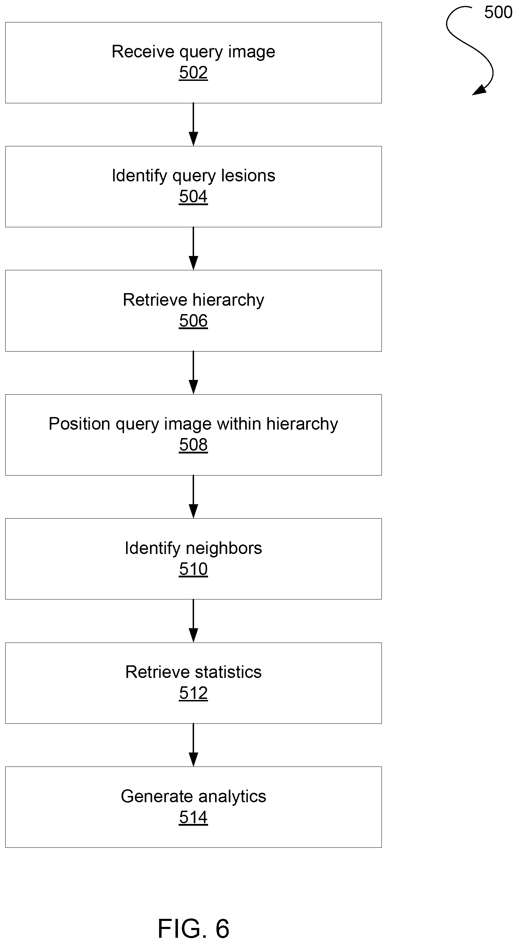

FIG. 6 illustrates a flowchart of an example method 500 for using the analysis and visualization system to evaluate a query image. FIG. 7 illustrates a block diagram of the example method 500 of FIG. 6. FIGS. 6 and 7 are described concurrently.

At 502, a query image is received. For example, a clinician examining an image taken on a patient may submit the entire image as a query to the system.

At 504, the query image may be processed to identify query lesions in the query image and characteristics of the lesions. In examples, the clinician may identify a lesion or another one or more ROIs and submit the particular lesion or ROI to the system for analysis. The lesion is processed through an inference engine , such as a trained deep learning neural network, to extract the features.

At 506, the structured lesion database is retrieved. The structured lesions database may refer to structured lesion database 114 of FIGS. 1, 2, and 4. The full contents of the database may be retrieved, or a defined subset, such as any of the task related databases 214a-e of FIG. 2. A pre-generated archive may be used, a personalized archive, a combination of a pre-generated and a personalized archive, a combination of the personalized archives of multiple users, or a newly generated subset database based on parameters entered by the user may be used to populate the structured lesion database. Because the data structure may be scaled to user selections, it may be configured to accommodate a single user, or a network of many users, or allow the users to combine their data and share the jointly learned structured lesion database. If desired, databases, data sets, or particular cases may be subtracted from the process.

In examples, an inference-based approach may be used to populate the database and form the hierarchical data structure. Using previously trained representation, new data may be populated into an already structured database without retraining the models.

At 508, the query lesion is positioned within the lesion database. Based on the features extracted by the inference engine , a mapping engine orients the query lesion within the structured lesion database. Once the query lesion is placed inside the structured lesion database, its relationship to all the other lesions arranged in the structured lesion database can be explored.

At 510, neighbors of the query lesion are identified. At 512, statistics associated with the neighbors are retrieved. The query lesion may be compared with its neighbors. For example, some neighbors may have known findings (e.g., a biopsy outcome) that may be used to evaluate potential outcomes for the query lesion. The query lesion may be compared with its neighboring lesions or, in other examples, with its non-neighboring lesions which may be at different stages, that may be used to evaluate the potential staging of the query lesion.

At 514, visualization based on the statistics and the relationships are generated. As can be seen in FIG. 7, the steps of method 500 can be performed in multiple orders. In FIG. 7, an image of the structed data map 514 and the query lesion's position within the map in presented prior retrieving the neighbors' statistics 512.

The visualization may be generated by default or in response to a user selection. FIG. 7 presents an example of the visualization in the form of a horizontal network graph 550. The display may show how the structured lesion database clusters into different sub-groups. The query lesion 552 can be traced from the mass category 554 to the oval-shape category 556 and finally the indistinct-margin category 558. The clinician may view and compare the query lesion with similar neighboring lesions at 510. Additionally, the user may view the statistics of the neighbors 512 revealing, for example, how many lesions were recalled, biopsied, or identified as malignant. These may assist the clinician in making a well-informed diagnostic or therapeutic decision based on previous similar cases.

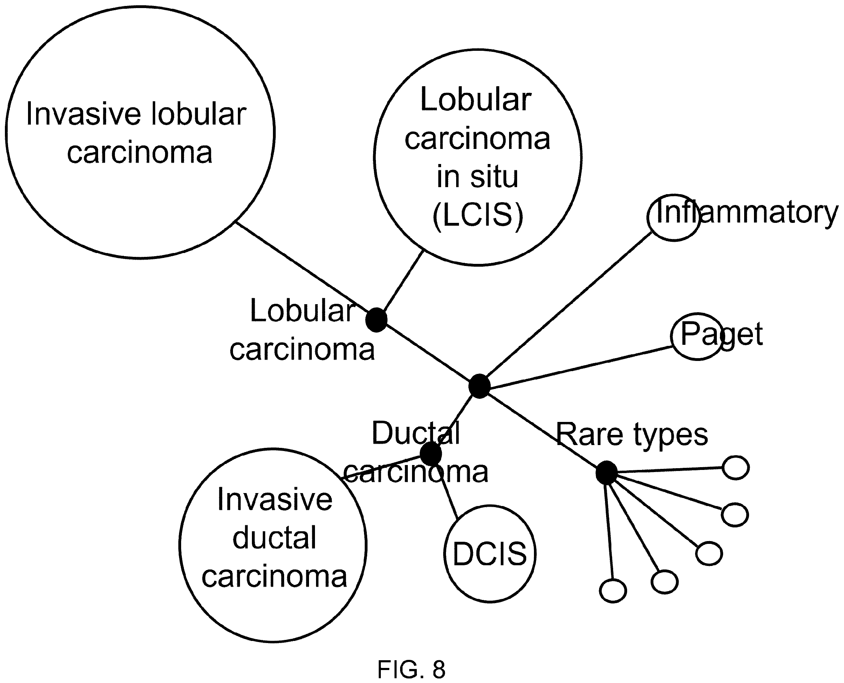

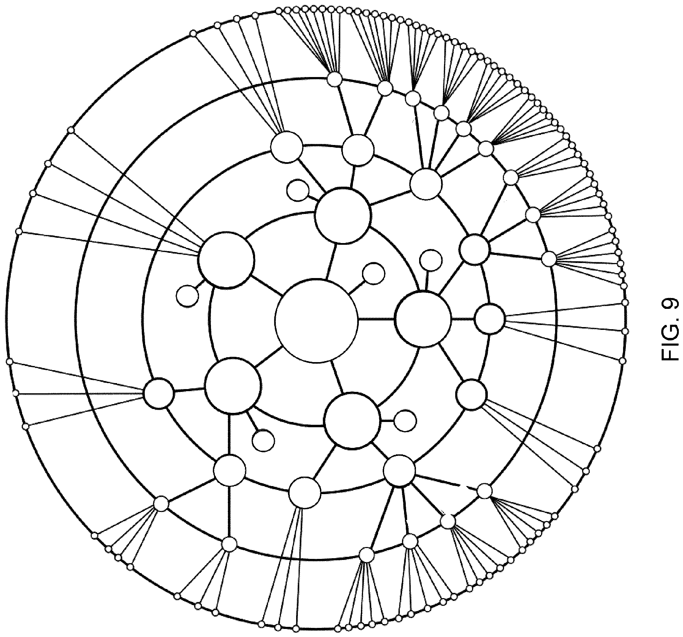

Other examples of visualizations are contemplated, including various hierarchical graphs depicted in two- or three dimensions. FIG. 8 illustrates another example visualization in the form of a hierarchical cluster tree. FIG. 9 illustrates another example visualization in the form of a radial graph.

A clinician or other user may be able to more effectively evaluate the query lesion based on the neighboring. For example, visualizations may include other modality imaging information, such as MRI, or ultrasound, and comparisons may predict how the query lesion may appear in the other different modalities which may in turn reveal additional diagnostically significant information about the query lesion. The query lesion can be visualized along an axis of time progression as compared with neighboring benign or malignant lesions, and evaluated for a possible trajectory of the query lesion based on past and future scans of the neighboring lesions.

Visualizations may be fully or partially interactive. A user may select a particular presentation of the visualization. A clinician may selection, such as with a mouse click, one or more of the displayed lesions or groups to access associated scans or other images. The user may also make adjustments to the database once the hierarchical graph is displayed, to see changes based on the images populated into the data structure.

A user may discover lesion characteristics in relation to other lesions in the same category such as by comparing and exploring neighboring lesions with respect to different characteristics. Examples of characteristics may include recall information, lesion biopsy outcomes, BIRADS score, etc. These characteristics may be selected to appear within the visualization or may be accessed via an operation, such as a menu or a popup. A user may explore the lesion "timeline," comparing the query lesion with earlier and/or later scans of neighboring lesions, which may indicate the possible trajectory for the query lesion. A user may explore image generation models, which may model a predicted future appearance of the query lesions, such as through selection of a time point or a slider bar. A user may compare the query lesions with multimodality data, such as by viewing images of neighboring lesions taken with alternate imaging modalities such as ultrasound or MRI. A user may explore image generation models, which may model the appearance of the query lesion as imaged by an alternate imaging modality. In examples, a user may also select whether or not a particular query lesion, or one or more query lesions, may be added to the structured lesion database. In some embodiments, further to being added to the structured lesion database, a query lesion may be used to update one or more models used on association with the lesion database.

FIG. 10 illustrates a flowchart of an example method 600 of defining inference engines based on clinical tasks. Together with task-specific lesion databases, such as databases 214a-e of FIG. 4, automated learning modules can be defined for learning tasks defined according to clinical tasks. Different learning tasks will generate different learned feature representations and may change image and/or lesion distribution with the data structure according to the differences. Through definition of different learning tasks, the structure and arrangement of the structured lesion database can be influenced through the orientation of the axis of the structure, or the principal distribution of data within the structure.

At 602, a first classifier is trained. For example, a deep learning (DL) neural network classifier may be trained to sort a set of lesion data based on lesions being benign or malignant. Here, the clinical task is diagnosis of malignant lesions requiring intervention by the clinician and the learning task is the identification of features which may be used to identify a malignant lesion. Generating a statistical graph based on the learning of the first classifier will result in a structure where malignant and benign lesions occupy two ends of the feature distribution, regardless of how similar lesions may appear. This task forms a feature distribution along an axis running from benign to malignant.

Using a large-scale database and the first classifier, the large-scale database can be classified along the benign-malignant axis. Using this classified database, the DL neural network can be trained to differentiate benign lesions from malignant lesions. This differentiating network can be used to extract information from any number of lesion databases and form a set of characteristics or features that define benign lesions as compared to malignant lesions.

At 604, a second classifier is trained. For example, another DL neural network may be trained to organize lesions based on whether a pair of lesions are similar or dissimilar. The resulting statistical graph will group similar lesions together based on overall appearance, like margin, size, shape, etc. The primary axis for the structure produced by the second classifier is the lesions' appearance. Since no malignancy information is considered, it may group together similar looking benign and malignant lesions. Training of the first and second classifier may be interchanged.

At 606, a first joint classifier is trained. In the present example, the first joint classifier is trained to find similar lesions and learn malignancy simultaneously. This joint task-training may produce a structure where similar lesions are close together unless they differ in whether they are benign or malignant. Similar lesions with different degrees of malignancy may have more distance from each other or occupy different sections of the structure.

At 608, a third classifier is trained. For example, another DL network classifier may be trained to learn different attributes or sematic information about a lesion, such as lesion shape, size, spiculations, surrounding densities, etc. The third classifier may produce a semantically meaningful structure that separates lesions along multiple axes or dimensions by their different attributes. A visual semantic network may be suitable for this learning/training task to learn visual attributes and characteristics.

At 610, a second j oint classifier is trained. In the present example, the second joint classifier may combine the tasks of all of the first, second, and third classifier. The second joint classifier may be trained to differentiate benign and malignant tumors, trained to find similar lesions, and trained to identify different lesion attributes or semantic information. The first, second, and third classifiers may be combined to form a bigger model to perform all three tasks simultaneously. The resulting data structure will be formed along the multiple axes: along lesion malignancy (benign or malignant), along lesion similarity (similar or not similar), and along lesion attributes (margin, morphology, shape, size, density, etc.). Further tasks can be added or combined in different ways as needed. Together with suitable deep learning neural network models, the resulting structured lesion graphs may then be re-formed further along the new task axis.

While a general order for the steps of the method 600 is shown in FIG. 10, those of skill in the art will understand the method 600 can include more or fewer steps or can arrange the order of the steps differently than the example order of the flowchart shown in FIG. 10. In embodiments, steps of the method 600 can be removed, new steps may be added, and steps may be exchanged, or otherwise reordered. For example, any number of classifiers (first, second, third, etc.) may be trained until total number of single classifiers are trained, before any joint classifiers are trained. In other examples, a first number of single classifiers may be trained and a second number of subsets of the single classifiers selected for training a matching number of joint classifiers (the second number), with any number of additional single or joint classifiers trained afterwards.

Once the structured lesion data graph is formed, a user may visualize or interact with it in a number of ways. A user may select a certain axis of the data structure and visualize the lesions along that axis. For example, a user may choose to visualize lesions distributed along a biopsy outcome axis, and see how lesions morph, change and are related to each other moving from the end of an axis running from benign lesions to malignant lesions. A user may visualize a lesion stage progression axis, and see how a stage 1 lesion can progress or change, and be related to the other lesions gradually into the late stage 1, stage 2, stage 3 and stage 4.

In examples, a user may select multiple axes of the lesion data structure, and visualize the lesions that are distributed along the joint direction of those multiple axes. For example, one can visualize the lesions distributed along the biopsy outcome axis together with the lesion margin axis, and see how lesions morph or change, and are related to each other moving from the benign lesions with smooth and regular margins, to the other end of malignant lesions with less smooth and more irregular margins. A user may select one, two, three or more axis/axes.

A user may choose to project the lesion universe into a lower dimension space for better visualization and interaction. For example, a user may choose three axes and project the lesion data structure into a 3-dimensional space, then visualize the lesion data structure, such as by panning, rotating, or magnifying it to see how all the different lesions are distributed throughout this 3-dimensional space.

A user may use a color-map to color lesions in the lesion data structure. Features that are not along the axis of the universe may be highlighted. For example, by coloring benign lesions with colors closer to the green, and coloring malignant lesions with colors closer to the red. This may aide the user in appreciating the distribution of the benign lesions as compared with the distribution of the malignant lesions

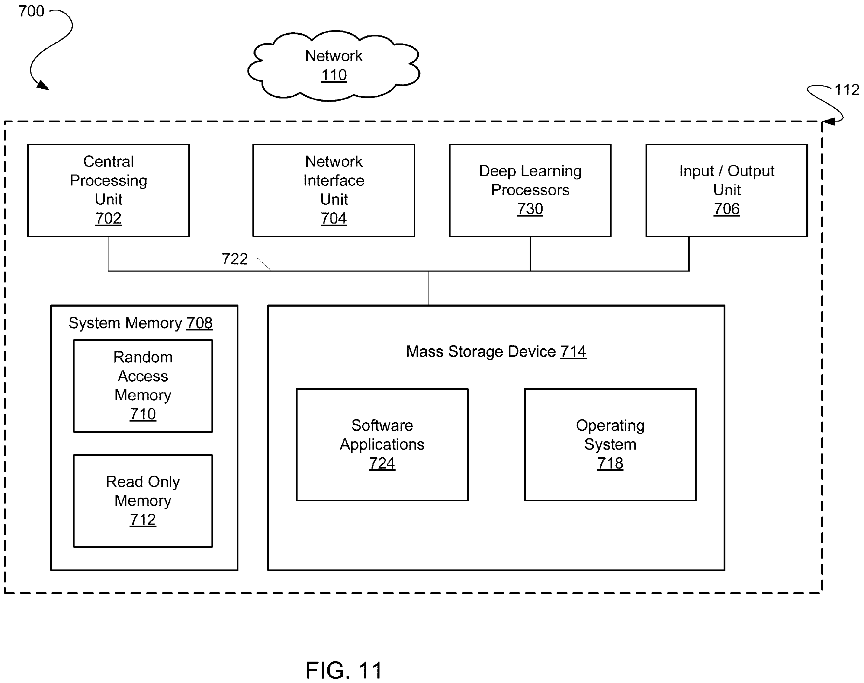

FIG. 11 illustrates an example block diagram of a virtual or physical computing system 700. One or more aspects of the computing system 700 can be used to implement the systems and methods for medical image analysis and visualization described herein. In particular, the computing system 700 may be used to implement the medical image analysis server 112, the structured lesion database or repository server 114, or the one or more of the clinician computing devices 102a-n.

In the embodiment shown, the computing system 700 includes one or more processors 702, a system memory 708, and a system bus 722 that couples the system memory 708 to the one or more processors 702. The system memory 708 includes RAM (Random Access Memory) 710 and ROM (Read-Only Memory) 712. A basic input/output system that contains the basic routines that help to transfer information between elements within the computing system 700, such as during startup, is stored in the ROM 712. The computing system 700 further includes a mass storage device 714. The mass storage device 714 is able to store software instructions and data for the implementation of systems, methods, and components of the present disclosure. The one or more processors 702 can be one or more central processing units or other processors.

The mass storage device 714 is connected to the one or more processors 702 through a mass storage controller (not shown) connected to the system bus 722. The mass storage device 714 and its associated computer-readable data storage media provide nonvolatile, non-transitory storage for the computing system 700. Although the description of computer-readable data storage media contained herein refers to a mass storage device, such as a hard disk or solid state disk, it should be appreciated by those skilled in the art that computer-readable data storage media can be any available non-transitory, physical device or article of manufacture from which the central display station can read data and/or instructions.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid state memory technology, CD-ROMs, DVD (Digital Versatile Discs), other optical storage media, magnetic cassettes, magnetic tape, magnetic disk storage or other magnetic storage devices, or any other medium which can be used to store the desired information and which can be accessed by the computing system 700.

According to various embodiments of the invention, the computing system 700 may operate in a networked environment using logical connections to remote network devices through the network 110. The network 110 may correspond to network 110 of FIG. 1, and is a computer network, such as an enterprise intranet and/or the Internet. The network 110 can include a LAN, a Wide Area Network (WAN), the Internet, wireless transmission mediums, wired transmission mediums, other networks, and combinations thereof. The computing system 700 may connect to the network 110 through a network interface unit 704 connected to the system bus 722. It should be appreciated that the network interface unit 704 may also be utilized to connect to other types of networks and remote computing systems. The computing system 700 also includes an input/output controller 706 for receiving and processing input from a number of other devices, including a touch user interface display screen, or another type of input device. Similarly, the input/output controller 706 may provide output to a touch user interface display screen or other type of output device.

As mentioned briefly above, the mass storage device 714 and the RAM 710 of the computing system 700 can store software instructions and data. The software instructions include software applications 724 and an operating system 718 suitable for controlling the operation of the computing system 700. The mass storage device 714 and/or the RAM 710 also store software instructions, that when executed by the one or more processors 702, cause one or more of the systems, devices, or components described herein to provide functionality described herein, including generating of the medical image analysis and visualization system, execution of the disclosed methods, and support of user interactions with the system. For example, the mass storage device 714 and/or the RAM 710 can store software instructions that, when executed by the one or more processors 702, cause the computing system 700 to receive and execute managing network access control and build system processes.

Software modules, such as software applications 724, may be executed by deep learning processors 730. Deep learning processors 730 can be graphics processing units (GPUs) or field-programmable gate arrays (FPGAs). Deep learning processors 730 can be hosted by a deep learning cloud platform.

Referring to the figures and examples presented herein generally, the disclosed environment provides a physical environment with which aspects of the outcome prediction and trade-off analysis systems can be implemented. Having described the preferred aspects and implementations of the present disclosure, modifications and equivalents of the disclosed concepts may readily occur to one skilled in the art. However, it is intended that such modifications and equivalents be included within the scope of the claims which are appended hereto.

### Examples:

Illustrative examples of the systems and methods described herein are provided below. An embodiment of the system or method described herein may include any one or more, and any combination of, the clauses described below.
Clause 1. A method of analysis for medical image data, the method comprising: receiving an image of breast tissue; identifying at least one region of interest (ROI) in the image based on one or more tissue characteristics associated with ROI; defining a query image as a subset of the image of breast tissue including the ROI; retrieving a hierarchy of lesion data including a plurality of images of breast tissue, the hierarchy being formed based on one or more relationships among the plurality of images; positioning the query image within the hierarchy of lesion data based on the one or more tissue characteristics; determining a position of the query image within the hierarchy of the lesion database; identifying one or more neighbor images of the query image from among the plurality of images based on the position; retrieving statistics associated the one or more neighbor images; generating analytics associated with the query image based on the statistics; generating a graphic depicting the analytics; and displaying the graphic. The method according to any of the clauses is preferably a computer-implemented method.
Clause 2. The method of analysis for medical image data of any other clause, wherein the hierarchy of lesion data is retrieved based on a selection received from a user.
Clause 3. The method of analysis for medical image data of any other clause, wherein the hierarchy of lesion data is based on a pre-generated archive.
Clause 4. The method of analysis for medical image data of any other clause, wherein the hierarchy of lesion data is based on an image generated neural network.
Clause 5. The method of analysis for medical image data of any other clause, wherein the hierarchy of lesion data is based on a personalized archive associated with the one or more users.
Clause 6. The method of analysis for medical image data of any other clause, wherein the selection comprises parameters defining a subset of the plurality of images.
Clause 7. The method of analysis for medical image data of any other clause, wherein the hierarchy of lesion data includes the subset of the image containing the ROI.
Clause 8. The method of analysis for medical image data of any other clause, wherein the hierarchy of lesion data further comprises one or more of patient information, semantic characteristics, and analytical characteristics.
Clause 9. The method of analysis for medical image data of any other clause, wherein the hierarchy comprises a high dimensional hierarchical statistical graph.
Clause 10. The method of analysis for medical image data of any other clause, wherein the one or more relationships are based on a first set of features used to classify the plurality of medical images.
Clause 11. The method of analysis for medical image data of any other clause, wherein the first set of features defines a first axis upon which the hierarchy is structured.
Clause 12. The method of analysis for medical image data of any other clause, further comprising a second set of features defining a second axis; wherein the hierarchy is structured upon both the first axis and the second axis.
Clause 13. The method of analysis for medical image data of any other clause, further comprising a second set of features used to classify the plurality of medical images, wherein classifications based on the second set of features are indicated using a range of colors.
Clause 14. The method of analysis for medical image data of any other clause, wherein the graphic is generated based on a selection received from a user, wherein the selection indicates one or more of a horizontal network graph, a radial graph, a hierarchical cluster tree, a two-dimensional graphic, a three-dimensional graphic, and a graphic depicting on a subset of hierarchy.
Clause 15. The method of analysis for medical image data of any other clause, wherein the graphic of the analytics includes one or more of the hierarchy of lesion data, the one or more neighbor images, and the statistics.
Clause 16. The method of analysis for medical image data of any other clause, further comprising generating a time progression of the query image based on the one or more neighbor images.
Clause 17. The method of analysis for medical image data of any other clause, further comprising generating a projection mapping a trajectory of the query ROI's through a multi-dimension space.
Clause 18. The method of analysis for medical image data of any other clause, wherein the query image is associated with a first imaging modality, and the method further comprises generating a depiction of the query image associated with a second imaging modality based on the one or more neighbor images.
Clause 19. The method of analysis for medical image data of any other clause, wherein the time progression or the image from the second imaging modality is generated using an image generation neural network.
Clause 20. The method of analysis for medical image data of any other clause, wherein the statistics comprise one or more of: a recalled number of lesions in the one or more neighbor images, a biopsied number of lesions in the one or more neighbor images, a malignant-identified number of lesions in the one or more neighbor images, a margin measurement, a shape measurement, a risk measurement, a density measurement, a size measurement, a characterization of morphology, a characterization of pathology, and a characterization of functional properties including at least elasticity, stiffness, and angiogenesis.
Clause 21. A computer system for medical image analysis, comprising: one or more processors; and non-transitory computer-readable storage media encoding instructions which, when executed by the one or more processors, causes the computer system to create: a database of lesion data, wherein the lesion data comprises a plurality of images of breast tissue, each image of the plurality of images arranged into a hierarchical structure; an inference engine configured to: apply a trained model to represent each region of interest by a high dimensional feature representation, and arrange the plurality of images into the hierarchical structure based on the high dimensional feature representation of each region of interest and one or more relationship among the plurality of images determined based on the feature representation; a mapping engine configured to: project the high dimensional feature representation to a lower dimensional representation; and to identify one or more neighbors of a query image based on the lower dimensional representation and the hierarchy.
Clause 22. The computer system for medical image analysis of any other clause, wherein the one or more neighbors are identified based on a distance measurement.
Clause 23. The computer system of any other clause, further comprising a visualization engine configured to generate a graphic depicting the hierarchy.
Clause 24. The computer system of any other clause, wherein the graphic is configured as a training tool.
Clause 25. The computer system of any of any other clause, wherein the inference engine is a deep learning engine.
Clause 26. The computer system of any of any other clause, wherein the database of lesion data comprises one or more of: a resource pool of medical images, patient information, and breast lesion images and associated semantic/analytical characteristics.
Clause 27. The computer system of any other clause, wherein the database comprises a plurality of breast lesion image data constructed as a high dimensional hierarchical statistical graph.
Clause 28. A method of generating a high dimensional lesion database for analysis for medical image data, the method comprising: training a first neural network to sort a plurality of medical images based on a degree of malignancy associated with each image; training a second neural network to sort the plurality of medical images based on a degree of similarity among two or more images of the plurality of medical images; training a third neural network to sort the plurality of medical images based on one or more characteristics associated with each image; and generating an interactive graphic depicting at least a portion of the plurality of medical images in a hierarchy based on at least one of the first, second, and third neural networks.
Clause 29. The method of any other clause, wherein the second neural network is at a dual-input deep neural network.
Clause 30. The method of any other clause, wherein the third neural network is a semantic neural network model.
Clause 31. The method of any other clause, further comprising training a fourth neural network to sort the plurality of medical images based on segmentation and measurement; and wherein the hierarchy is further based on the fourth neural network.
Clause 32. The method of any other clause, wherein the first, second, third, and/or fourth model are trained as one system to extract semantical/analytical characteristics.
Clause 33. The method of any other clause, wherein two or more of the first, second, third, and/or fourth model are be combined using a multitask neural network to yield different types of information about a region of interest from one network.
Clause 34. The method of any other clause, further comprising training a fifth neural network to determine a future appearance of a query image based on the hierarchy.
Clause 35. The method of any other clause, further comprising training the fifth neural network to perform a comparison between a current image and an associated prior image for each image of the plurality of medical images; and sort the plurality of medical images based on the comparison.
Clause 36. The method of any other clause, further comprising receiving a selection of one or more of the first, second, and third neural networks; and wherein the hierarchy is based on the selection.
Clause 37. The method of any other clause, further comprising receiving a selection indicating the portion of the plurality of medical images to be depicted in the interactive graphic.
Clause 38. A computer system for medical image analysis, comprising: one or more processors; and non-transitory computer-readable memory comprising instructions which, when executed by the one or more processors, causes the computer system to create: an inference engine programmed to provide analytical insights about a query region of interest in an image of breast tissue and compare population distribution across sites and characterize population level statistics; a mapping engine programmed to pull relevant information in the form of reports, future scans, etc. about neighborhood regions of interest; and an interaction and visualization engine programmed to provide diagnostic decision support and aid in knowledge discovery.

This disclosure described some examples of the present technology with reference to the accompanying drawings, in which only some of the possible examples were shown. Other aspects can, however, be embodied in many different forms and should not be construed as limited to the examples set forth herein. Rather, these examples were provided so that this disclosure was thorough and complete and fully conveyed the scope of the possible examples to those skilled in the art.

Although specific examples were described herein, the scope of the technology is not limited to those specific examples. One skilled in the art will recognize other examples or improvements that are within the scope of the present technology. Therefore, the specific structure, acts, or media are disclosed only as illustrative examples. Examples according to the technology may also combine elements or components of those that are disclosed in general but not expressly exemplified in combination, unless otherwise stated herein. The scope of the technology is defined by the following claims and any equivalents therein.

## Claims

1. A computer-implemented method of analysis for medical image data, the method comprising:
receiving an image of breast tissue;
identifying at least one region of interest (ROI) in the image based on one or more tissue characteristics associated with ROI;
defining a query image as a subset of the image of breast tissue including the ROI;
retrieving a hierarchy of lesion data from a lesion database including a plurality of images of breast tissue, the hierarchy being formed based on one or more relationships among the plurality of images;
positioning the query image within the hierarchy of lesion data based on the one or more tissue characteristics, preferably by determining a position of the query image within the hierarchy of the lesion database;
identifying one or more neighbor images of the query image from among the plurality of images based on the position;
retrieving statistics associated the one or more neighbor images;
generating analytics associated with the query image based on the statistics;
generating a graphic depicting the analytics; and
displaying the graphic.

2. The computer-implemented method of analysis for medical image data of claim 1, wherein the hierarchy of lesion data:
- is based on a pre-generated archive; and/or
- is based on an image generated neural network; and/or
- is based on a personalized archive associated with the one or more users.

3. The computer-implemented method of analysis for medical image data of claim 1, wherein the hierarchy of lesion data is retrieved based on a selection received from a user, wherein the selection comprises parameters defining a subset of the plurality of images.

4. The computer-implemented method of analysis for medical image data of any of claims 1-3, wherein the hierarchy of lesion data includes the subset of the image containing the ROI.

5. The computer-implemented method of analysis for medical image data of any of claims 1-4, wherein the hierarchy of lesion data further comprises one or more of patient information, semantic characteristics, and analytical characteristics.

6. The computer-implemented method of analysis for medical image data of any of claims 1-5, wherein the hierarchy comprises a high dimensional hierarchical statistical graph.

7. The computer-implemented method of analysis for medical image data of any of claims 1-6, wherein the one or more relationships are based on a first set of features used to classify the plurality of medical images.

8. The computer-implemented method of analysis for medical image data of claim 7, wherein the first set of features defines a first axis upon which the hierarchy is structured,
preferably further comprising a second set of features defining a second axis, wherein the hierarchy is structured upon both the first axis and the second axis.

9. The computer-implemented method of analysis for medical image data of claim 8, further comprising a second set of features used to classify the plurality of medical images, wherein classifications based on the second set of features are indicated using a range of colors.

10. The computer-implemented method of analysis for medical image data of any of claims 1-9, wherein the graphic is generated based on a selection received from a user, wherein the selection indicates one or more of a horizontal network graph, a radial graph, a hierarchical cluster tree, a two-dimensional graphic, a three-dimensional graphic, and a graphic depicting on a subset of hierarchy.

11. The computer-implemented method of analysis for medical image data of any of claims 1-10, wherein the graphic of the analytics includes one or more of the hierarchy of lesion data, the one or more neighbor images, and the statistics,
wherein preferably the time progression or the image from the second imaging modality is generated using an image generation neural network.

12. The computer-implemented method of analysis for medical image data of any of claims 1-11, further comprising generating a time progression of the query image based on the one or more neighbor images,
wherein preferably the time progression or the image from the second imaging modality is generated using an image generation neural network.

13. The computer-implemented method of analysis for medical image data of any of claims 1-12, further comprising generating a projection mapping a trajectory of the query ROI's through a multi-dimension space,
wherein preferably the time progression or the image from the second imaging modality is generated using an image generation neural network.

14. The computer-implemented method of analysis for medical image data of any of claims 1-13, wherein the query image is associated with a first imaging modality, and the method further comprises generating a depiction of the query image associated with a second imaging modality based on the one or more neighbor images,
wherein preferably the time progression or the image from the second imaging modality is generated using an image generation neural network.

15. The computer-implemented method of analysis for medical image data of any of claims 1-14, wherein the statistics comprise one or more of: a recalled number of lesions in the one or more neighbor images, a biopsied number of lesions in the one or more neighbor images, a malignant-identified number of lesions in the one or more neighbor images, a margin measurement, a shape measurement, a risk measurement, a density measurement, a size measurement, a characterization of morphology, a characterization of pathology, and a characterization of functional properties including at least elasticity, stiffness, and angiogenesis.
